# EUROPÄISCHE PATENTSCHRIFT

(11) **EP 1 003 559 B1**
(45) Veröffentlichungstag und Bekanntmachung des Hinweises auf die Patenterteilung: **27.02.2002**
(21) Anmeldenummer: 98942616.8
(22) Anmeldetag: 24.07.1998
(51) Int. Cl.: A61K 47/48

(54) **IMMOBILISIERUNG VON VITAMIN-A-SÄURE DURCH KATIONISCHE POLYELEKTROLYTE**
IMMOBILIZATION OF VITAMIN A ACID BY CATIONIC POLYELECTROLYTES
IMMOBILISATION D'ACIDE DE VITAMINE A PAR DES POLYELECTROLYTES CATIONIQUES

(30) Priorität: 25.07.1997 DE 19732139
(43) Veröffentlichungstag der Anmeldung: 31.05.2000
(73) Patentinhaber: Max-Planck-Gesellschaft zur Förderung der Wissenschaften e.V., 80539 München (DE)
(72) Erfinder: THÜNEMANN, Andreas, D-12207 Berlin (DE)
(74) Vertreter: Böhm, Brigitte, Dipl.-Chem. Dr.
(86) Internationale Anmeldenummer: EP9804644
(87) Internationale Veröffentlichungsnummer: WO9904821

(56) Entgegenhaltungen:
- WO-A-96/15810
- DE-A- 4 428 641
- US-A- 4 999 348
- THUENEMANN ANDREAS: "Immobilization of Retinoic Acid by Cationic Polyelectrolytes" LANGMUIR, Bd. 13, Nr. 23, 12. November 1997, Seiten 6040-6046, XP002091048
- ANTONIETTI M ET AL: "Polyelectrolyte-Surfactant Complexes: A New Class of Highly Ordered Polymer Materials" TRENDS IN POLYMER SCIENCE, Bd. 5, Nr. 8, August 1997, Seite 262-267 XP004086001
- ANTONIETTI M ET AL: "MESOMORPHOUS POLYELECTROLYRE-SURFACTANT COMPLEXES" ADVANCED MATERIALS, Bd. 7, Nr. 8, 1. August 1995, Seiten 751-753, XP000519833
- ANTONIETTI, M. ET AL.: "Complexation of Lecithin with Cationic polyelectrolytes: " Plastic Membranes" as Models for the Structure of the Cell Membrane ?" LANGMUIR, Bd. 11, Nr. 7, 1995, Seiten 2633-2638, XP002091049 in der Anmeldung erwähnt

## Beschreibung

Die vorliegende Erfindung betrifft mesomorphe Komplexe aus Vitamin A-Säure und kationischen Polyelektrolyten, insbesondere in Form von Filmen oder Nano-Dispersionen, Verfahren zur ihrer Herstellung sowie die Verwendung der mesomorphen Komplexe als Vitamin A-Substituat zur Herstellung von Arzneimitteln.

Vitamin A-Säure ist ein hochkristallines niedermolekulares Material. Lipophile Hormone, wie Vitamin A-Säure, Steroide, Thyroidhormone und Vitamin D₃ wirken durch Bindung mit Ligand-aktivierten Transkriptionsfaktoren, umfassend die Steroid/nuclear receptor Superfamilie (R.M. Evans, Science 240 (1988), 889). Derzeit wird intensiv die Rolle von Vitamin A-Säure bei der Zelldifferenzierung durch Untersuchung der Bindungseigenschaften der Retinoide an spezifische Proteine untersucht (W. Bourguet, M. Ruff, P. Chambon, H. Gonemeyer und D. Moras, Nature 375 (1995), 377; J.-P. Renaud, N. Rochel, M. Ruff, V. Vivat, P. Chambon, H. Gronemeyer und D. Moras, Nature 378 (1995), 681).

Zusätzlich zu ihrer wichtigen Rolle bei der Weiterleitung pleiotroper Effekte auf Morphogenese, Differenzierung und Hämostase während der embryonalen und postnatalen Lebensphase zeigt Vitamin A-Säure ein großes Potenzial als pharmakologischer Wirkstoff. Derzeit wird Vitamin A-Säure bei der äußerlichen Behandlung von schweren Fällen der Akne verwendet, und ihre Verwendung für Hautverjüngungskuren wird außerdem diskutiert (A.H. Lewin, M.E. Bos, F.C. Zusi, X. Nair, G. Whiting, Bouquin, G. Tetrault und F.I. Carroll, Pharm. Res 11 (1994), 192). Schließlich gibt es auch Hinweise auf eine Inhibition maligner Tumoren durch Retinoide (G. Zanotti, M.R. D'Acunto, G. Malpeli, C. Folli und R. Berni, Eur. J. Biochem. 234(2) (1995), 563; E.P. Jaeger, P.C. Jurs und T.R. Stouch, Eur. J. Med. Chem. 28(4) (1993), 275).

Alle Retinoide besitzen dieselben charakteristischen Eigenschaften als hoch UV-aktiver Chromophor und sind in wässrigem Medium schwer löslich und chemisch instabil. Deshalb binden in der Natur Retinoide an spezifische retinoidbindende Proteine, die Schutz, Löslichkeit und Transportierbarkeit in Körperflüssigkeiten vermitteln. In Bezug auf die Verabreichung von Vitamin A-Säure als pharmakologischer Wirkstoff ist ein Hauptproblem die nötige Immobilisierung. Eine Möglichkeit, eine derartige Immobilisierung und dadurch einen Schutz der Vitamin A-Säure zu erreichen, ist, sie an ein Protein zu binden, wie dies von der Natur demonstriert wird. Ein erfolgreiches Beispiel für diese Strategie wurde bei Zanotti et al. gezeigt, der Transthyretin und Vitamin A-Säure kokristallisiert hat. Diese Vorgehensweise ist jedoch schwierig und kostenintensiv.

In dem europäischen Patent 0 680 748 A1 wird eine Zusammensetzung in Form eines Gels offenbart, die ein saures, hydrophiles Medium und mindestens einen Gelbildner, der aus einem vernetzten kationischen Polymer gebildet ist, enthält, die dadurch gekennzeichnet ist, dass das hydrophile Medium ein Medium ist, das eine Menge an organischem Lösungsmittel enthält, die 20 bis 90 % des Gesamtgewichts der Zusammensetzung beträgt, und eine Wassermenge enthält, die höchstens 45 % des Gesamtgewichts der Zusammensetzung beträgt, wobei der Gelbildner der Zusammensetzung ein makroskopisch homogenes Aussehen eines Gels und Stabilität verleiht, sowie die kosmetische Verwendung dieses Gels insbesondere zur Hautdepigmentierung. Derartige Gele weisen eine amorphe Struktur auf, wobei ihre Viskosität und damit auch ihre Stabilität durch den Vernetzungsgrad der Polyelektrolyte bestimmt wird. Die Freisetzung einer in diesem Gel in ungebundener Form vorliegenden Substanz, wie z.B. Retinoat, kann über die Einstellung der Viskosität des Gels reguliert werden.

Nachteilig ist der hohe Gehalt an organischem Lösungsmittel in diesem Gel bei einer Anwendung als Arzneimittel.

Es war daher die Aufgabe der vorliegenden Erfindung, eine Möglichkeit zur Immobilisierung von Vitamin A-Säure bereitzustellen, die leicht und möglichst kostengünstig durchzuführen ist.

Gelöst wird diese Aufgabe gemäß vorliegender Erfindung durch die Bereitstellung mesomorpher Komplexe aus Vitamin A-Säure und kationischen Polyelektrolyten.

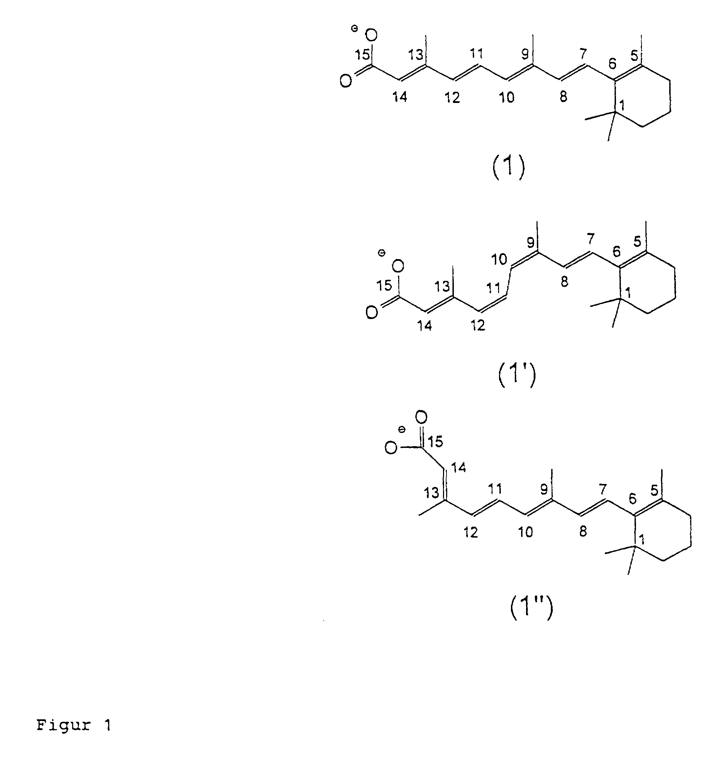

Die Komplexierung der Vitamin A-Säure mit kationischen Polyelektrolyten basiert auf der Feststellung, dass die Bildung geordneter Strukturen in Lösung oder in festem Zustand oft mittels Selbstorganisation erfolgt, indem ein oberflächenaktives Mittel an einen Polyelektrolyten anheftet. Die treibende Kraft für diesen Prozess sind elektrostatische und hydrophobe Wechselwirkungen in wässriger Lösung. Kürzlich wurde von E.A. Ponomarenko, A.J. Waddon, D.A. Tirrell und W.J. MacKnight, Langmuir 12 (1996), 2169; A. Ponomarenko, A.J. Waddon, K.N. Bakeev, D.A. Tirrell und W.J. MacKnight, Macromolecules 29 (1996), 4340 eine detaillierte Untersuchung über selbstorganisierte Komplexe von synthetischen Polypeptiden mit entgegengesetzt geladenen oberflächenaktiven Mitteln mit niedrigem Molekulargewicht veröffentlicht. Außerdem wurde gezeigt, dass die Komplexierung von oberflächenaktiven Mitteln mit Polyelektrolyten zu einer großen Anzahl von stabilen Mesophasen mit großer struktureller Vielfalt führt (M. Antonietti, J. Conrad und A. Thünemann, Macromolecules 27 (1994), 6007; M. Antonietti, S. Henke und A. Thünemann, Advanced Materials 8 (1996), 41; M. Antonietti, A. Kaul und A. Thünemann, Langmuir 11 (1995),2633). Es wurde weiter festgestellt, dass nicht nur synthetische oberflächenaktive Mittel, sondern auch amphiphile Verbindungen hierzu geeignet sein könnten. Vitamin A-Säure ist aufgrund des Vorhandenseins der Carboxylfunktion zum einen polar und aufgrund der Anwesenheit der hydrophilen Kopfgruppe und des langen Kohlenwasserstoffteils zum anderen hydrophob (Figur 1), also eine amphiphile Verbindung.

Im Rahmen der vorliegenden Erfindung wurden vorzugsweise drei verschiedene Polyelektrolyten für die Komplexierung von Vitamin A-Säure verwendet. Zum einen wurde PDADMAC (Poly(dimethyldiallylammoniumchlorid) verwendet, das dafür bekannt ist, dass es mit natürlichen Lipiden stabile lösliche Komplexe (M. Antonietti, A. Kaul und A. Thünemann, Langmuir 11 (1995), 2633; M. Antonietti, A. Wenzel und A. Thünemann, Langmuir 12 (1996), 2111) bildet und supramolekular geordnete Gele mit Natriumdodecylsulfat bildet (F. Yeh, E.L. Sokolov, A.R. Khokhlov und B. Chu, J. Am. Chem. Soc. 118 (1996), 6615). Somit ist ein Komplex aus Vitamin A-Säure mit PDADMAC erfindungsgemäß besonders bevorzugt.

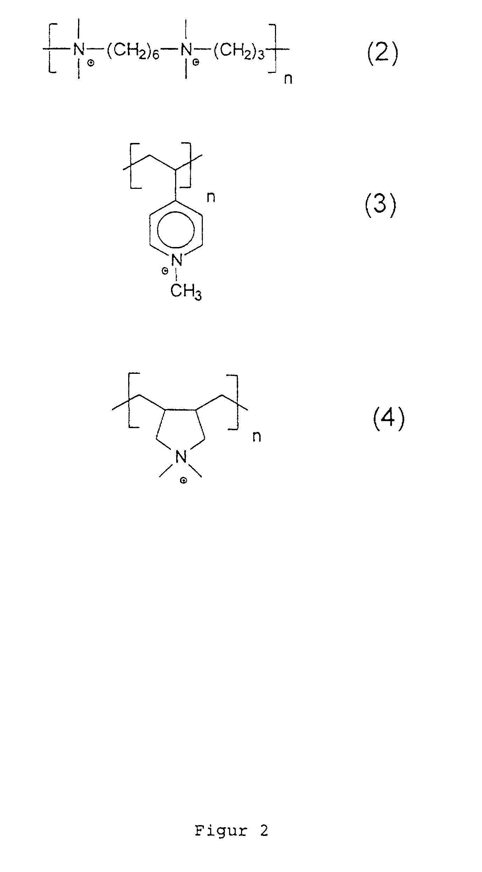

Weitere besonders bevorzugte, strukturell unterschiedliche kationische Polyelektrolyte, die für die Komplexierung im Rahmen der vorliegenden Erfindung besonders geeignet sind, sind PM4VP, Poly(N-methyl-4-vinylpyridinchlorid), ein Polyelektrolyt mit Ladungen auf den Seitengruppen (B. Philipp, W. Dawydoff und K.-J. Linow, Z. Chem. 22 (1982), 1) sowie Poly(ionen-6,3) mit den positiven Ladungen direkt auf der Polymerhauptkette (Figur 2), wobei PM4VP als "pendant type"-Polyelektrolyt und Poly(ionen-6,3) als sogenannter "integral type"-Polyelektrolyt bezeichnet wird. Im Hinblick auf seine Ladungen nimmt PDADMAC eine Zwischenstellung zwischen PM4VP und Poly(ionen-6,3) ein. Deshalb wird PDADMAC als "intermediate type"-Polyelektrolyt bezeichnet. Besonders bevorzugt ist außerdem die Verwendung von Polyethylenimin, erhältlich von BASF, Ludwigshafen, Deutschland, das unter den Markenbezeichnungen Lupasol vertrieben wird.

Als Polyelektrolyte werden weiterhin besonders bevorzugt Poly-L-Aminosäuren, insbesondere Poly-L-arginin, Ply-L-histidin, Poly-L-lysin oder ein Gemisch davon, verwendet. Durch die Wahl des kationischen Polyelektrolyten kann das Freisetzungsverhalten der in dem Komplex enthaltenen Vitamin A-Säure wie gewünscht eingestellt werden.

In den erfindungsgemäßen Komplexen können die Vitamin A-Säure und der kationische Polyelektrolyt in verschiedenen Verhältnissen vorliegen, wobei ein Verhältnis von 1 : 1 besonders bevorzugt ist. Die erfindungsgemäßen Komplexe lassen sich auch leicht zu filmartigen Gebilden verarbeiten, sodass sie in Form eines viskoelastischen Films vorliegen, welche interessante physikalische Eigenschaften aufweisen. Im Gegensatz zu relativ brüchiger kristalliner Vitamin A-Säure sind Komplexe mit Polyelektrolyten stark deformierbare viskoelastische Materialien. Diese erfindungsgemäßen Materialien zeigen lamellare Strukturen.

In einer besonders bevorzugten Ausführungsform liegen die erfindungsgemäßen Komplexe als Nano-Dispersion zusammen mit einem Dispergierhilfsmittel als Teilchen vor, wobei der Teilchendurchmesser < 5000 nm beträgt. In dieser erfindungsgemäßen Nano-Dispersion können alle dem Fachmann bekannten und gebräuchlichen Dispergierhilfsmittel verwendet werden, wobei Poloxamer 188 bevorzugt ist.

Die Mengenverhältnisse des Komplexes und des Dispergierhilfsmittels können variiert werden, um eine Nano-Dispersion mit jeweils gewünschten Eigenschaften, wie z.B. Partikelgröße, Vitamin A-Säure-Freisetzungsverhalten etc., zu erhalten. Das Verhältnis von Komplex zu Dispergierhilfsmittel beträgt vorzugsweise 1 : 10 bis 10 : 1, besonders bevorzugt 1 : 2 bis 2 : 1, und am meisten bevorzugt ist der Komplex und das Dispergierhilfsmittel in gleichen Mengen in der Nano-Dispersion enthalten.

Der Teilchendurchmesser der Nano-Dispersion wird je nach den Anwendungsanforderungen entsprechend ausgewählt. Er beträgt vorzugsweise 200 bis 5000 nm, vorzugsweise 250 bis 3000 nm, besonders bevorzugt 300 bis 2000 nm und am meisten bevorzugt 350 bis 1500 nm. In einer weiteren bevorzugten Ausführungsform sind die Teilchendurchmesser 350 bis 400 nm, besonders bevorzugt 350 bis 390 nm und am meisten bevorzugt 1 350 bis 1460 nm. Es zeigte sich überraschenderweise, dass die erfindungsgemäße Nano-Dispersion nicht nur für extrakorporale, sondern auch für intravenöse Anwendungen geeignet ist.

Die erfindungsgemäßen Komplexe und insbesondere PDADMAC-Retinoat, Poly(ionen-6,3)-Retinoat und PM4VP-Retinoat sind in einer Vielzahl von polaren organischen Lösungsmitteln, wie Methanol, Ethanol, 2-Butanol, Isopropanol und Chloroform löslich. In polaren Lösungsmitteln dissoziieren Polyelektrolytkomplexe mit oberflächenaktiven Mitteln sehr wahrscheinlich zumindest teilweise (M. Antonietti, S. Förster, M. Zisenis und J. Conrad, Macromolecules 28 (1995), 2270), wogegen in Lösungsmitteln niedriger Polarität solche Komplexe assoziiert bleiben können (K. Bakeev, S.a. Chugunov, I. Teraoka, W.J. MacKnight, A.B. Zezin und V.A. Kabanov, Macromolecules 27 (1994), 3926). Die Löslichkeit der bevorzugten erfindungsgemäßen Komplexe ist in Übereinstimmung mit kürzlich veröffentlichten Untersuchungen über Komplexe, welche aus konventionellen synthetischen Polyelektrolyten und gegensätzlich geladenen oberflächenaktiven Mitteln bestehen (M. Antonietti, J. Conrad und A. Thuenemann, Macromolecules 27 (1994), 6007).

Es hat sich gezeigt, dass die erfindungsgemäßen Komplexe überraschenderweise ohne Vernetzungsmittel mechanisch stabil sind, wobei die Stabilität des erfindungsgemäßen Komplexes variabel einstellbar ist. Damit kann in vorteilhafter Weise die Freisetzungskinetik der Vitamin A-Säure aus dem Komplex gezielt gesteuert werden und an die jeweiligen Anwendungserfordernisse angepasst werden. Als besonders günstig hat sich herausgestellt, dass die Komplexe mesomorph mit lamellarer Struktur sind und in einer besonders bevorzugten Ausführungsform ein physikalischer Ordnungszustand existiert, der dem eines smektischen Flüssigkristalls entspricht.

Insbesondere im Vergleich zu den aus dem Stand der Technik bekannten Gelen, die amorph sind und deren chemisches Verhalten, insbesondere ihr Freisetzungsverhalten, von in ihnen enthaltenen Substanzen durch den Vernetzungsgrad bestimmt wird, weisen die erfindungsgemäßen Komplexe Vorteile, wie z.B. kostengünstige Herstellung, gute Lagerfähigkeit, einfache Verarbeitbarkeit und Anwendbarkeit etc., auf.

Auch sind nur geringe Mengen oder überhaupt keine organischen Lösungsmittel in den erfindungsgemäßen Komplexen enthalten, sodass die zunehmend als kritisch betrachtete Verwendung von organischen Lösungsmitteln, insbesondere bei pharmazeutischen Anwendungen, vermieden werden konnte.

Ein weiterer Gegenstand der vorliegenden Erfindung ist ein Verfahren zur Herstellung der erfindungsgemäßen Komplexe aus Vitamin A-Säure und kationischen Polyelektrolyten, bei dem man Lösungen von Vitamin A-Säure und einem Polyelektrolyten mischt und die gebildeten Rohkomplexe isoliert und gegebenenfalls nach an sich bekannten Methoden aufreinigt. In einer bevorzugten Ausführungsform der Erfindung verwendet man als Polyelektrolyten PDADMAC, PM4VP, Poly(ionen-6,3), Polyethylenimin oder Poly-L-Aminosäuren, insbesondere Poly-L-arginin, Poly-L-histidin, Poly-L-lysin oder ein Gemisch davon. In einer weiteren bevorzugten Ausführungsform arbeitet man bei dem erfindungsgemäßen Verfahren in basischer Lösung, vorzugsweise indem man Vitamin A-Säure in einer basischen wässrigen Lösung auflöst und dann eine wässrige Lösung des Polyelektrolyten zugibt, vorzugsweise tropfenweise. Die erfindungsgemäßen Komplexe fallen bei der Zugabe aus und können leicht abgetrennt werden. Beispielsweise kann die weitere Reinigung durch Wiederauflösung in Methanol erfolgen und überschüssige Vitamin A-Säure und Salz durch Ultrafiltration entfernt werden. Das erfindungsgemäße Verfahren wird üblicherweise bei Raumtemperatur, vorzugsweise bei 20°C bis 30°C, jedoch bei nicht mehr als 60°C bis 80°C, besonders bevorzugt ≤ 30°C durchgeführt.

Ein weiterer Gegenstand der vorliegenden Erfindung ist die Verwendung der erfindungsgemäßen mesomorphen Komplexe, insbesondere in Form von viskoelastischen Filmen oder Nano-Dispersionen, als Vitamin A-Substituat. Für alle Verwendungen von Vitamin A-Säure, bei der insbesondere die Instabilität der Säure von Nachteil war, können anstelle der reinen Säure nun die mesomorphen Komplexe verwendet werden, die bevorzugt als viskoelastische Filme, welche immobilisierte Vitamin A-Säure enthalten, und besonders bevorzugt als Nano-Dispersion vorliegen.

Besonders bevorzugt werden die erfindungsgemäßen Komplexe, insbesondere in Form von Filmen oder Nano-Dispersionen, als pharmazeutische Wirkstoffe verwendet, wobei insbesondere derzeit die Hauterkrankungen oder die Hemmung des Wachstums maligner Tumoren ein geeignetes und bevorzugtes Anwendungsgebiet darstellen.

Ein weiterer Gegenstand der vorliegenden Erfindung sind daher pharmazeutische Zusammensetzungen, welche die erfindungsgemäßen mesomorphen Komplexe, insbesondere in Form von viskoelastischen Filmen oder Nano-Dispersionen, der vorliegenden Erfindung enthalten. Derartige pharmazeutische Zusammensetzungen können überall dort eingesetzt werden, wo bisher Vitamin A-Säure oder andere Retinoide zum Einsatz kamen.

Die Komplexierung von Vitamin A-Säure mit strukturell verschiedenen kationischen Polyelektrolyten (z.B. integral, intermediate und pendant type) führt zur Bildung erfindungsgemäßer neuer Materialien mit interessanten strukturellen und optischen Eigenschaften ebenso wie zu neuen pharmazeutischen Zusammensetzungen. Ihre Haupteigenschaften sind:
1. Die neuen mesomorphen Komplexe enthalten bis zu 70 Gew.-% optisch aktive Moleküle. Aufgrund der starken chromophoren Wechselwirkungen im festen Zustand zeigen die Komplexe zusätzliche starke hochenergetische Absorption bei 252 nm. Darüber hinaus kann das Festphasen-UV-Visspektrum signifikant beeinflusst werden durch zusätzliche Chromophore, wie z.B. Methyl-4-vinylpyridin, das weitere Möglichkeiten der Veränderung der Absorptionscharakteristika eröffnet.
2. Die Komplexe können einfach zu Nano-Dispersionen oder zu Filmen mit unterschiedlichen lamellaren Strukturen verarbeitet werden, die morphologisch sehr ähnlich zu S_{A}-Flüssigkristallen sind.
3. Abhängig von der Polyelektrolytstruktur kann die Glasübergangstemperatur im Bereich zwischen -19 und 28°C eingestellt werden, und auch die mechanischen Eigenschaften sind in einem weiten Bereich variabel. Unter pharmazeutischem Gesichtspunkt können die Komplexe als neue Formulierung eines sehr aktiven Wirkstoffs angesehen werden. Es ist davon auszugehen, dass die Komplexe eine reduzierte Toxizität und auch eine reduzierte teratogene Wirkung aufweisen, verglichen zu klassischen Formulierungen enthaltend Vitamin A-Säure. Die Komplexe können verwendet werden zur Behandlung von Hautkrankheiten, wie z.B. Akne, Psoriasis und Hyperkeratosen. Die Formulierung der Komplexe als kolloide Partikel könnte ein weiterer Weg sein, das pharmazeutische Potenzial von Vitamin A-Säure z.B. als Wirkstoff bei der Inhibition des Wachstums maligner Tumoren auszunutzen. Vitamin A-Säure, welche ionisch an verschiedene Polyelektrolyten gebunden ist, ist außerdem ein vielversprechendes Material für biomimetische Anwendungen. Es kann davon ausgegangen werden, dass die Komplexe auch verwendet werden können als Teil eines photosynthetischen Systems, wobei Protonen von der Innenseite einer Membran auf die Außenseite transportiert werden, und dabei ein elektrochemischer Gradient gebildet wird, der vermutlich die Synthese von ATP fördert. Auf alle Fälle ist die optische Aktivität der natürlichen photosensitiven Pigmente von beträchtlichem Interesse, da sie sowohl Schlüsse auf die Protein-Chromophor-Wechselwirkung zulassen wird ebenso wie auf Konformationsänderungen, die nach der Lichtabsorption auftreten. Es ist davon auszugehen, dass durch eine Untersuchung der uniaxial ausgerichteten multilamellaren Komplexfilme das Verständnis der molekularen Basis der optischen Aktivität von Komplexen in natürlichen Systemen vorangetrieben werden kann.

Eine weitere Ausführungsform betrifft ein Verfahren zur Behandlung eines Patienten mit den erfindungsgemäßen Komplexen, die insbesondere als Film oder Nano-Dispersion vorliegen, der an Hauterkrankungen, insbesondere Akne, Psoriasis oder Hyperkeratosen, oder an malignen Tumoren leidet.

Die folgenden Beispiele sollen die Erfindung in Verbindung mit den Figuren weiter erläutern. Hierbei zeigt
Figur 1: die Konformationen der Vitamin A-Säure: all-trans (1), 1 1-cis (1') und 13-cis (1")
Figur 2: Polyelektrolyte, die verwendet werden zur Komplexierung: "integral type": Poly(ionen-6,3) (2), "pendant type": Poly(N-methyl-4-vinylpyridiniumchlorid) (3); "intermediate type": Poly(diallyldimethylammoniumchlorid) (4)

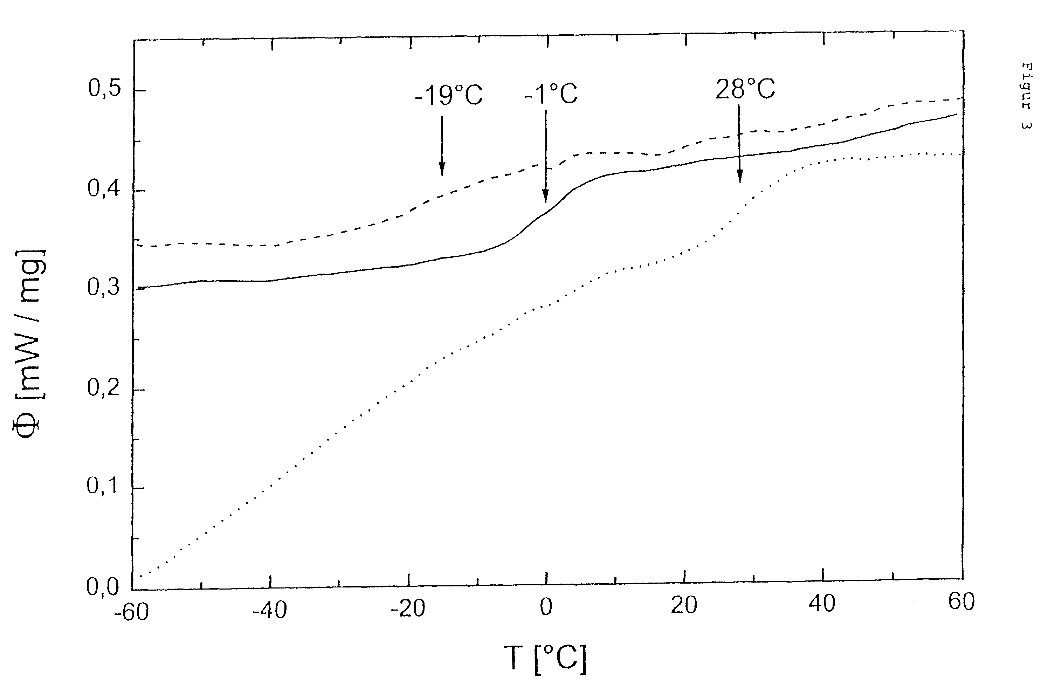

Figur 3 zeigt DSC-Kurven von PM4VP-Retinoat (gestrichelte Linie), Poly(ionen-6,3)-Retinoat (durchgezogene Linie) und PDADMAC-Retinoat (gepunktete Linie).
Figur 4 zeigt ein Spannungsdehnungsdiagramm von PDADMAC-Retinoat, das als Film vorliegt.
Figur 5 zeigt einen Polarisationsmikrograph eines PDADMAC-Retinoatfilms.
Figur 6 zeigt eine Weitwinkelröntgenstreuung eines PDADMAC-Retinoatfilms (obere Kurve) und Retinoatpulver (untere Kurve).
Figur 7 zeigt Kleinwinkelröntgenstreuungsdiagramme für (a) PM4VP-Retinoat, (b) Poly(ionen-6,3)-Retinoat und (c) PDADMAC-Retinoat.

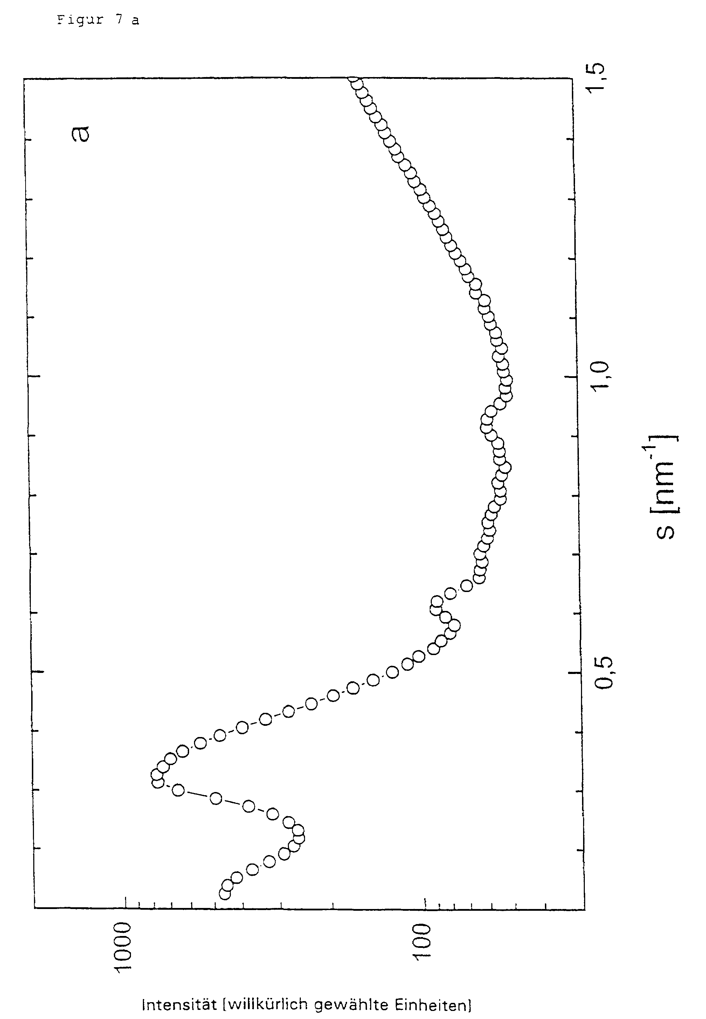

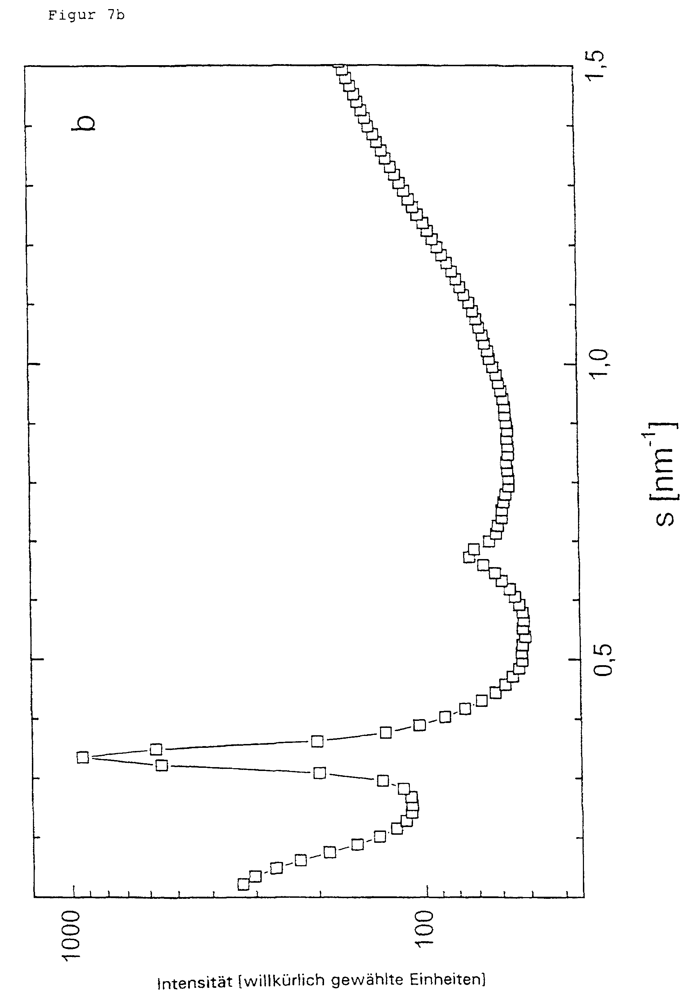

Die Teile a bis c der Figur 7 zeigen die Ergebnisse eines Streuungsexperiments bei kleinen Streuvektoren für die drei verschiedenen Komplexe. Im Diagramm von PM4VP-Retinoat wurden drei Peaks mit Abstandsverhältnissen 1 : 2 : 3 gefunden. Das Diagramm von Poly(ionen-6,3)-Retinoat zeigt zwei scharfe Reflektionen mit Abstandsverhältnissen 1 : 2 und das von PDADMAC-Retinoat hat eine scharfe und zwei schwache, breite Reflektionen mit Verhältnissen 1 : 2 : 3.

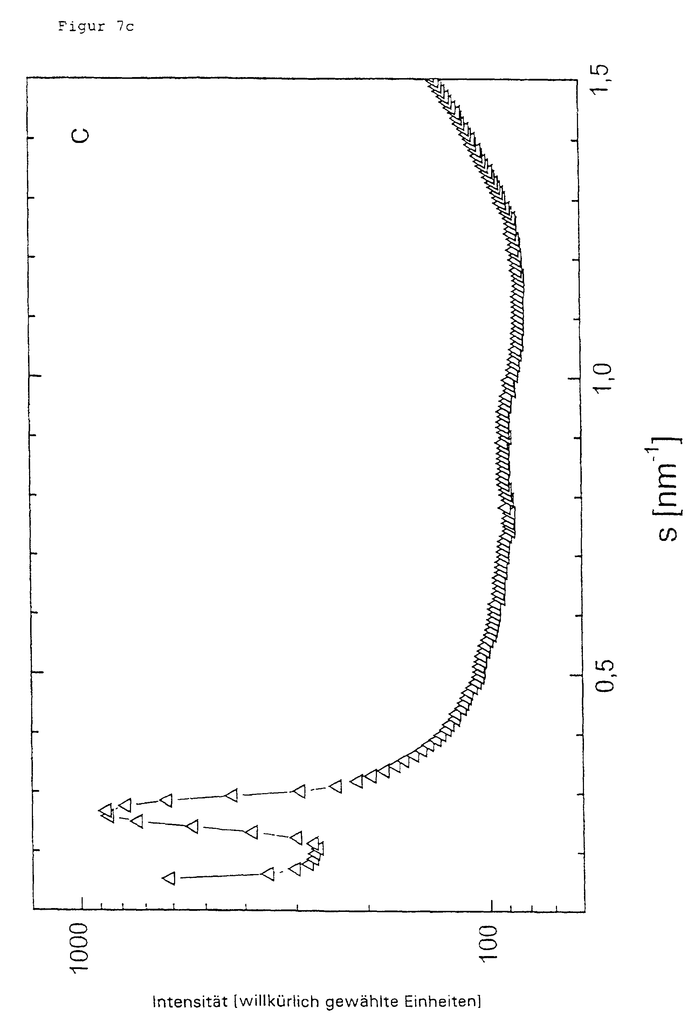

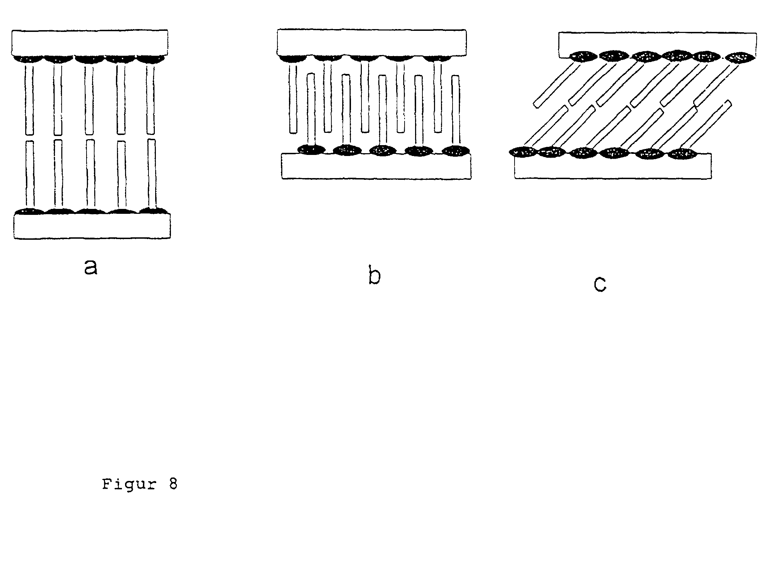

Figur 8 ist eine schematische Darstellung möglicher struktureller Anordnungen von Vitamin A-Polyelektrolyt-Komplexen. Teil a: PDADMAC-Retinoat; Teile b und c: Poly(ionen-6,3)-Retinoat und PM4VP-Retinoat.

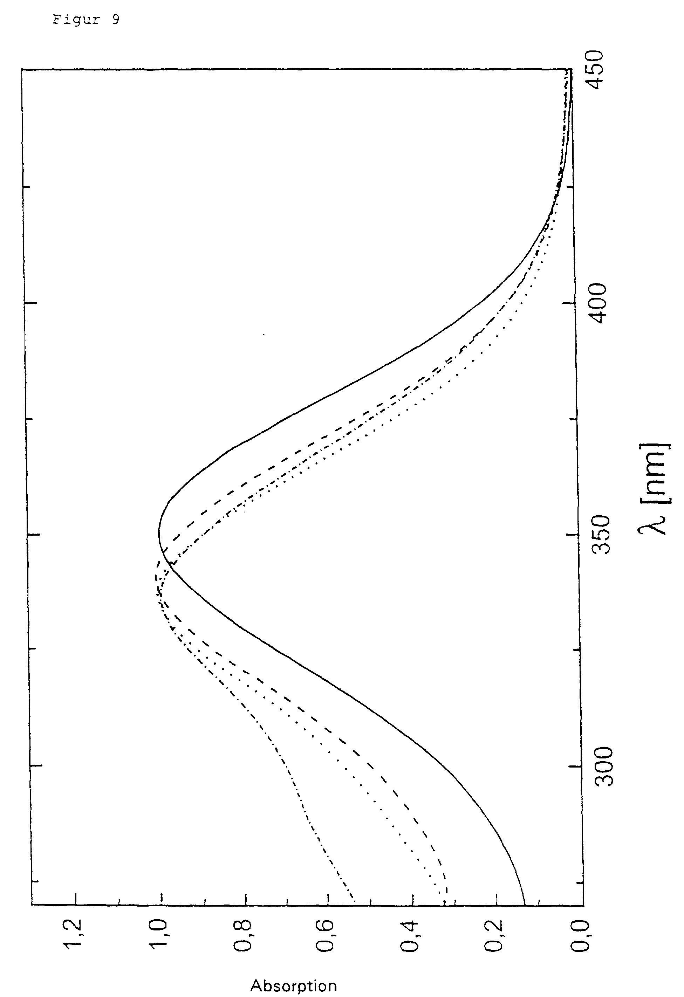

Figur 9 zeigt UV/Vis-Spektren von Vitamin A-Säurekomplexe in methanolischer Lösung: Vitamin A-Säure (durchgezogene Linie), PM4VP-Retinoat (gestrichelte Linie), Poly(ionen-6,3) (gepunktete Linie) und PDADMAC-Retinoat (gestrichelt-gepunktete Linie).

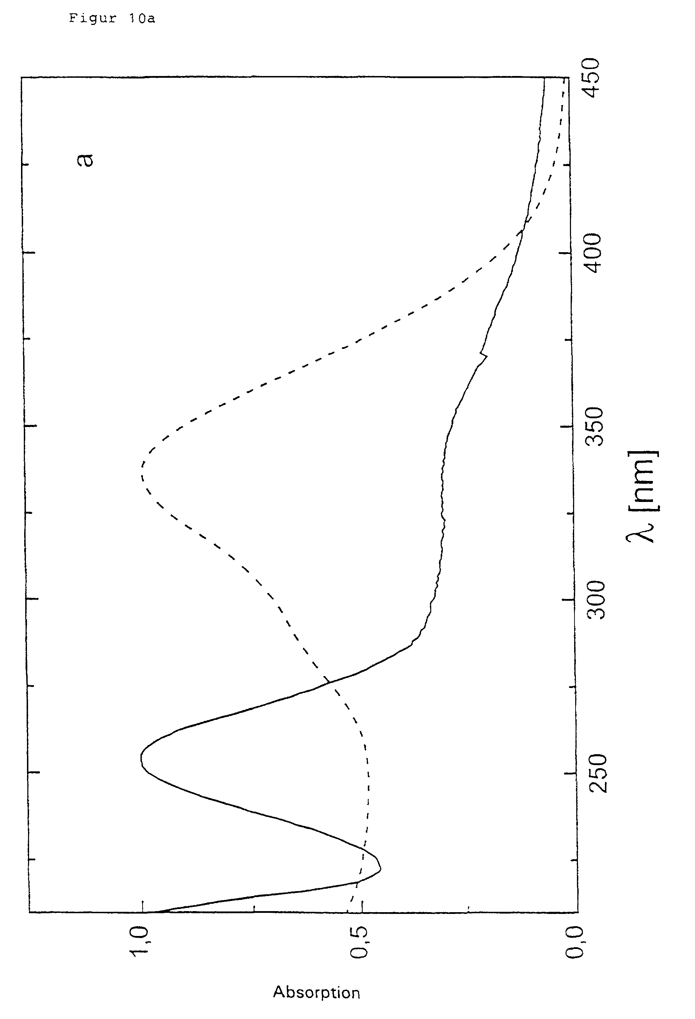

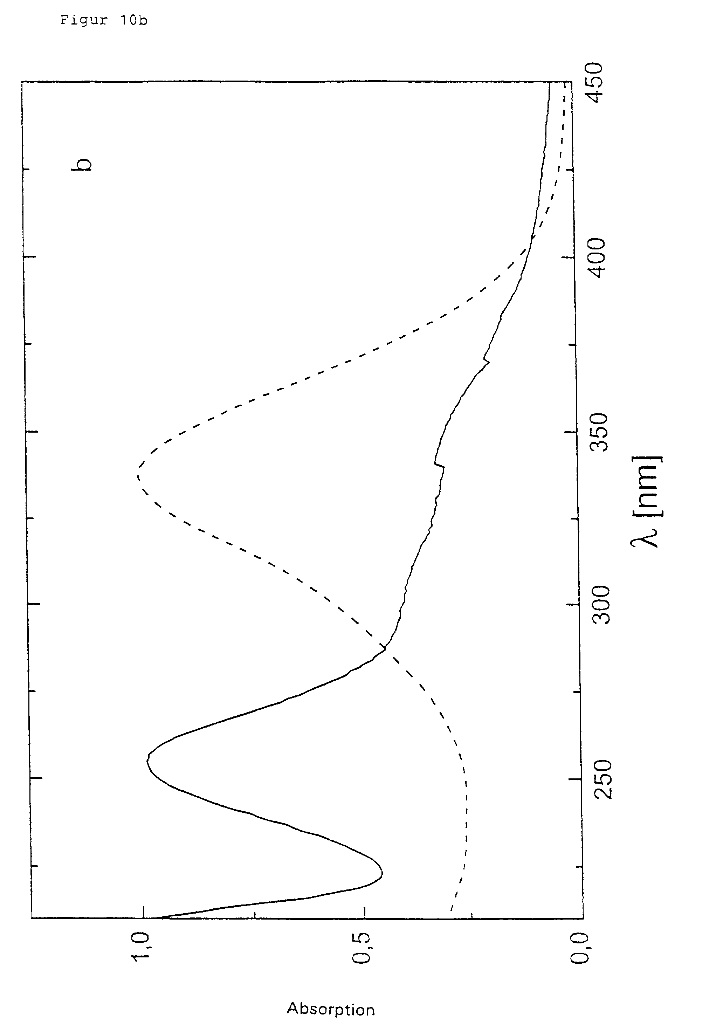

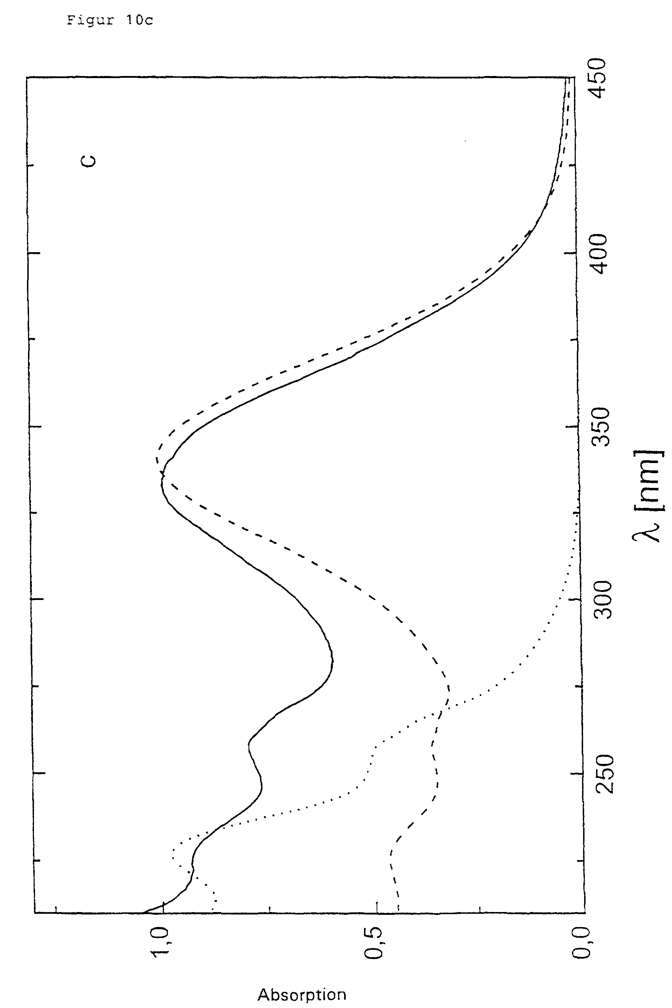

Figur 10 zeigt einen Vergleich der UV-Spektren von methanolischer Lösung (getrichelte Linie) und Filmen von Vitamin A-Säurekomplexen (durchgezogene Linie): PDADMAC-Retinoat (a), Polyionen-Retinoat (b), PM4VP-Retinoat (c).

### Beispiel 1

### Materialien

Kristalline all-trans Vitamin A-Säure (Tretionin) als Pulver, hochmolekulargewichtiges Poly(diallyldimethylammoniumchlorid) (20 w/w wässrige Lösung) und hochmolekulargewichtiges Poly(ionen-6,3bromid) wurden von Aldrich Chemical Co. erworben. Das Molekulargewicht von Poly(diallyldimethylammoniumchlorid) wurde durch Viskosimetrie in 0,5 N Natriumchloridsalzlösung festgestellt als Mᵥ = 180.000 g/mol. Das Ergebnis eines wässrigen GPC war M_{w} = 623.000 g/mol, Mₙ = 187.000 g/mol und die Lichtstreuung ergab einen Wert von 525.000 g/mol. Diese stark unterschiedlichen Werte weisen auf das allgemeine Problem der exakten Molekulargewichtsbestimmung von Polyelektrolyten hin. Jedoch ist die breite Molekulargewichtsverteilung und die Ungenauigkeit der Molekulargewichtsbestimmung für die Komplexbildung nicht weiter von Belang. Poly(N-methyl-4-vinylpyridiniumchlorid) wurde hergestellt aus Poly(4-vinylpyridin) über eine polymeranaloge Reaktion mit drei Äquivalenten Methyljodid in Nitromethan. Jodid wurde durch Chlorid ausgetauscht mittels Ultrafiltration unter Verwendung einer Natriumchloridlösung. Die Ausbeute der Methylierung wurde durch ¹H-NMR Spektroskopie als 100 % bestimmt. Das Poly(4-vinylpyridinchlorid) wurde hergestellt durch radikalische Polymerisationsreaktion in Lösung (R.M. Fouss, M. Wanatabe und B.D. Coleman, J. Polym. Sci. 48 (1960), 5). Sein Molekulargewicht wurde bestimmt durch THF-GPC als M_{w} = 180.000 g/mol. Für Poly(ionen-6,3chlorid) ist das vom Anbieter angegebene hohe Molekulargewicht etwas irreführend, da keine Molekulargewichte, welche höher sind als 30.000 bis 50.000 g/mol tatsächlich verfügbar sind. Für das Poly(ionen-6,3chlorid), welches in den vorliegenden Beispielen verwendet wurde, wurde das Molekulargewicht mittels Lichtstreuung als in der Größenordnung von 5.000 g/mol liegend festgestellt. Das Lösungsmittel für die Filmherstellung war HPLC gereinigtes Methanol (HPLC grade methanol (Aldrich Chemical Co.)).

### 1.1 Komplexbildung

100 mg Vitamin A-Säure wurden in wässriger Natriumhydroxidlösung aufgelöst und eine 0,5 %ige wässrige Lösung der Polyelektrolyte wurde tropfenweise unter Rühren zugegeben, bis keine weitere Präzipitation beobachtet wurde. Die erhaltenen Rohkomplexe wurden abgetrennt und in Methanol wieder aufgelöst. Mittels Ultrafiltration wurde überschüssige Vitamin A-Säure und Salz entfernt. Eine Elementaranalyse aller Komplexe zeigte, dass Natrium und Chlorid (bzw. Natrium und Bromid) zu weniger als 0,01 % anwesend waren. Freistehende Filme aller drei Komplexe wurden gegossen durch Ausgießen ihrer Lösungen in Methanol oder Ethanol auf Glasplatten. Die zweidimensionale Geometrie der Filme wurde durch Glasrahmen verschiedener Größe bestimmt, die auf der Glasplatte befestigt waren. Nach Verdampfen des Lösungsmittels bei 20°C verblieben Spuren des Lösungsmittels, die entfernt wurden im Vakuum bei Raumtemperatur über 24 Stunden.

### 1.2 Methoden

Weitwinkelröntgenstrahlenstreuungsuntersuchungen (WAXS) wurden ausgeführt mit einem Nonius PDS120 Pulverdiffraktometer in Transmissionsgeometrie. Ein FR590 Generator wurde verwendet als Quelle für Cu-Kα-Strahlung, Monochromatisierung des Primärstrahls wurde erreicht durch einen gekrümmten Ge-Kristall, die gestreute Strahlung wurde gemessen mit einem positionsempfindlichen Detektor CPS120. Die Auflösung dieses Detektors ist besser als 0,018°. Röntgenkleinwinkelstreukurven (SAXS) wurden aufgezeichnet mit einer Vakuumröntgenkamera mit "pinhole collimation" (Anton Paar, Österreich, Modell A-8054), welche ausgerüstet war mit Bildplatten (Typ BAS III, Fuji, Japan). Die Bildplatten wurden abgelesen mit einem MACScience Dip-Scanner IPR-420 und Pl reader DIPR-420 (Japan). DSC-Messungen wurden durchgeführt auf einem Netzsch DSC 200 (Deutschland). Die Proben wurden mit einer Aufheizrate von 10 K/min in zwei Aufheiz- und zwei Abkühlzyklen untersucht. Erste und zweite Zyklen waren im wesentlichen identisch. Dehnungsspannungsuntersuchungen wurden ausgeführt mit einem Materialtester der Firma Zwick mit der Bezeichnung Z010 (Deutschland). Optische mikroskopische Untersuchungen mit polarisiertem Licht auf dem Film wurden durchgeführt mit einem Zeiss DMRB Mikroskop (Deutschland). Die UV/Vis-Spektren wurden auf einem UVICON-Spektrophotometer 931 von Kontron Instruments aufgezeichnet. Simulierungen molekularer Anordnungen der Komplexe wurden durchgeführt unter Verwendung von Insight & Discover (BIOSYM Technologies, USA).

### Beispiel 2

### Dehnungsspannungsuntersuchungen

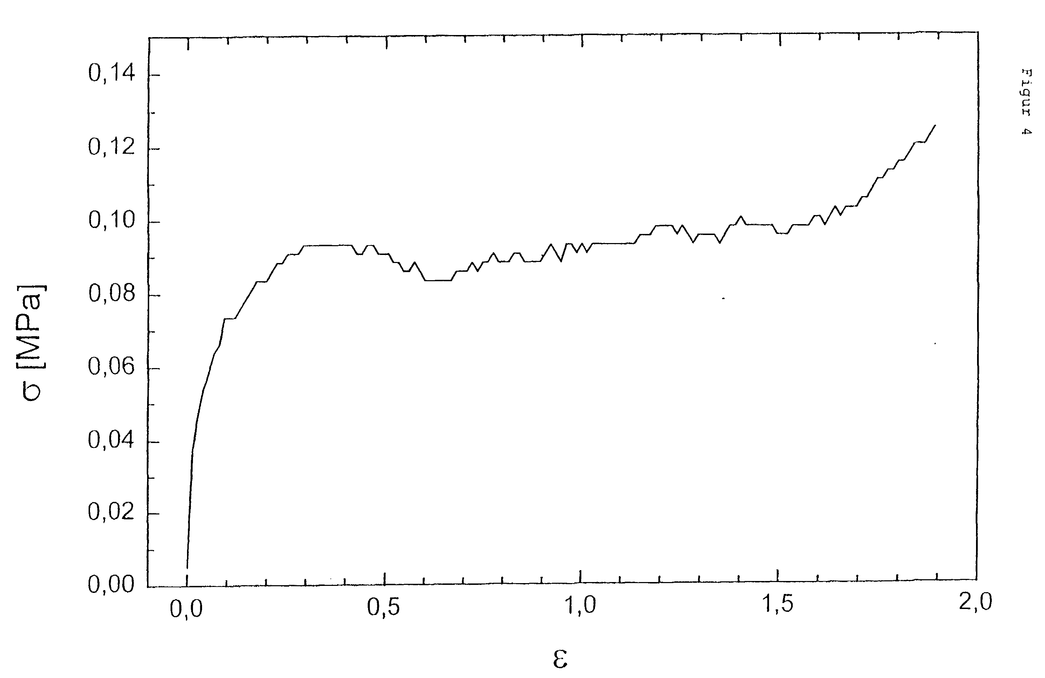

Aufgrund der höchsten Glasübergangstemperatur sind Filme, die aus PDADMAC-Retinoat gegossen wurden, mechanisch die stabilsten der Serie. Es war daher möglich, Spannungsdehnungsexperimente durchzuführen. Eine typische Spannungsdehnungskurve eines PDADMAC-Retinoatfilms ist in Figur 4 gezeigt. Das Spannungsdehnungsverhalten ist ähnlich zu dem, das typischerweise für gummiartiges Material beobachtet wird. Bei einer Dehnung von 1 % wurde das Zugfestigkeitsmodul von PDADMAC-Retinoat bestimmt als 4 MPa. Im Bereich zwischen 30 und 150 % wurde eine Dehnung bei konstanter Zugspannung beobachtet. Während weiterer Dehnung steigt die Spannung bis zu einem Maximalwert von 0,125 MPa. Das Material reisst bei einer Dehnung von 200 %. Die Ausbildung eines derartig flexiblen Films, der vorwiegend aus starren, stäbchenförmigen Molekülen besteht, ist bemerkenswert. Verantwortlich hierfür sind Interaktionen auf molekularem Niveau und vor allem Coulombkräfte zwischen Retinoateinheiten und den Polyelektrolyten, wobei eine Umwandlung von brüchigen Kristallen zu viskoelastischen Polymeren stattfindet.

### Beispiel 3

### Optische Mikroskopie

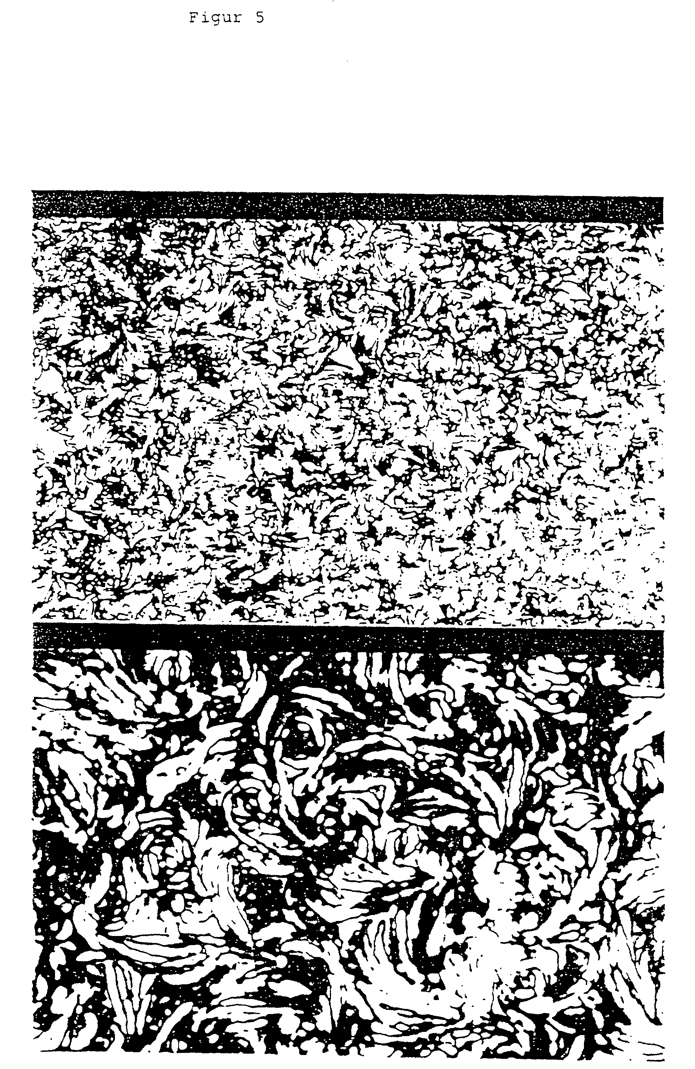

Die Filme aller Komplexe sind optisch anisotrop, wie während einer Untersuchung zwischen gekreuzten Polarisatoren festgestellt wurde. Ein Beispiel der optischen Textur ist in Figur 5 gezeigt. Offensichtlich sind die Komplexe mesomorphe Materialien, eine unzweifelhafte Identifikation der Mesophase an der Basis der optischen Textur ist nicht möglich.

### Beispiel 4

### Röntgenstreuung

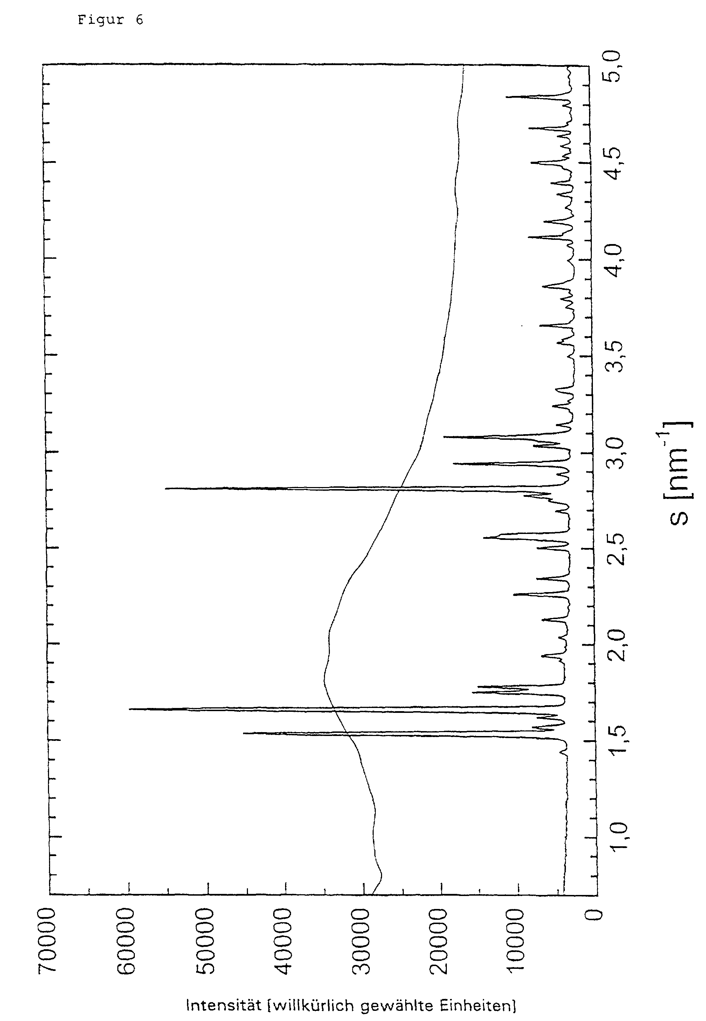

Das Fehlen von scharfen Reflexen im Weitwinkelapparat belegt, dass die drei bevorzugten Retinoatkomplexe tatsächlich amorph sind. Die Diagramme der drei Komplexe sind im wesentlichen identisch. Als ein Beispiel ist die WAXS-Kurve von PDADMAC in Figur 6 gezeigt. Die Streukurve zeigt einen charakteristischen amorphen Halo entsprechend einem Bragg-Abstand von ungefähr 0,52 nm. Dieser Wert ist erheblich größer als jene, die beobachtet werden für eine amorphe Packung von gesättigten Alkylketten in Komplexen von oberflächenaktiven Mitteln mit niedrigem Molekulargewicht mit synthetischen Polypeptiden (0,45 nm) (A. Ponomarenko, A.J. Waddon, K.N. Bakeev, D.A. Tirrell und W.J. MacKnight, Macromolecules 29 (1996), 4340) oder jener, die beobachtet wird für Polystyrolsulfonat-oberflächenaktives Mittel-Komplexe (0,43 nm) (M. Antonietti, J. Conrad und A. Thuenemann, Macromolecules 27 (1994), 6007). Die niedrigere Maximalposition des amorphen Halo im Retinoatkomplex verglichen mit jener, die beobachtet wird in Komplexen mit gesättigten Alkylketten, deutet darauf hin, dass der durchschnittliche Atomabstand im Vorhergenannten erheblich größer ist. Dies ist zu erwarten aufgrund des ausladenden Hexenrings und der daran konjugierten Alkyleneinheit des Retinoats, weshalb die Moleküle nicht amorph mit derselben Dichte zusammenlagern können wie flexible Ketten. Freie Vitamin A-Säure hat eine starke Tendenz zu kristallisieren (Figur 6) und zwei ähnliche kristalline Modifikationen der Vitamin A-Säure sind bekannt (eine trikline und eine monokline) (C.H. Stam, Acta Cryst. B28 (1972), 2936). Wie in Figur 6 gezeigt, ist die Fähigkeit von Vitamin A-Säure zu kristallisieren, stark unterdrückt durch die Komplexierung mit einem Polyelektrolyt. Die Komplexe bleiben über mehrere Monate amorph und es kann daraus geschlossen werden, dass sie thermodynamisch stabil sind.

Die Teile a bis c der Figur 7 zeigen die Ergebnisse eines Streuexperiments bei kleinen Streuvektoren für die drei verschiedenen Komplexe. Im Diagramm von PM4VP-Retinoat wurden drei Peaks mit Abstandsverhältnissen 1 : 2 : 3 gefunden. Das Diagramm von Poly(ionen-6,3)-Retinoat zeigt zwei scharfe Reflexe mit Abstandsverhältnissen 1 : 2 und das von PDADMAC-Retinoat hat eine scharfe und zwei schwache, breite Reflexe mit Verhältnissen 1 : 2 : 3.

### Beispiel 5

### UV/Vis-Spektroskopie

Der starke Einfluss der Komplexierung auf die optischen Eigenschaften kann am besten durch einen Vergleich der UV/Vis-Absorptionsspektra der Komplexe in einem Film in methanolischer Lösung gezeigt werden (Tabelle 1). Das UV/Vis-Spektrum der reinen all-trans Vitamin A-Säure in Methanol zeigt nur einen breiten Peak mit einem Maximum von 348 nm. Die Spektren von wiederaufgelösten Komplexfilmen sind sehr ähnlich zu dem von reiner all-trans Vitamin A-Säure. Nur eine geringe hypsochrome Verschiebung im Bereich von Δλₘₐₓ = 8 nm (PM4VP Retinoat) bis 12 nm (PDADMAC Retinoat) wurde festgestellt (Figur 9). Hieraus wurde geschlossen, dass in der Lösung keine signifikante Chromophorwechselwirkung stattfindet und die Retinoateinheiten sich wie isolierte Chromophore benehmen. Unter der Annahme, dass die hypsochrome Verschiebung als eine qualitative Messung für die Bindungskonstante dient, wurde die folgende Reihe für eine steigende Bindungsfestigkeit erhalten: PM4VP-Retinoat < Poly(ionen-6,3)-Retinoat < PDADMAC-Retinoat. Diese Abfolge ist in Übereinstimmung mit den DSC-Resultaten, in denen steigende Glasübergänge festgestellt wurden in derselben Reihenfolge.

Das Absorptionsverhalten der Komplexe in Filmen ist unterschiedlich zu dem in Lösung (Figur 10a-c): Das Spektrum von festem PDADMAC-Retinoat zeigt ein zusätzliches Absorptionsmaximum bei 252 nm, das stärker ist als das zweite bei 319 nm. Verglichen zu der Lösung wird für das letztere eine zusätzliche hypsochrome Verschiebung von Δλₘₐₓ = 17 nm beobachtet im Film. Ein sehr ähnliches Spektrum wie für PDADMAC-Retinoat wurde gefunden für Poly(ionen-6,3)-Retinoatfilme (Figur 10b): Charakteristisch ist hier das Absorptionsmaximum bei 253 nm. Wiederum ist das Maximum erheblich stärker als die zweite Absorptionsbande bei 297 nm. Diese zeigt wiederum eine zusätzliche hypsochrome Verschiebung von Δλₘₐₓ = 40 nm verglichen zu dem UV/Vis des Komplexes in Lösung.

Das Spektrum von PM4VP-Retinoatfilmen ist stärker strukturiert: Drei Maxima wurden festgestellt (Figur 10 c). Die zusätzlichen Absorptionsbanden sind zurückzuführen auf die UV-Aktivität der quaternären Vinylpyridiniumeinheit. Im Gegensatz zu den Spektren der beiden anderen Komplexe ist ein Maximum bei höherer Wellenlänge (334 nm) herausragend. Das Spektrum zeigt ein zweites Maximum bei 262 nm mit einer Schulter auf der Seite höherer Wellenlängen und ein drittes Maximum bei 226 nm. Die niedrige Intensität der höher energetischen Absorption im Bereich von 250 bis 270 nm von PM4VP-Retinoat im Vergleich zu Absorption bei kurzen Wellenlängen der anderen beiden Komplexe wird zurückgeführt auf den starken Einfluß des N-Methylpyridiniumchromophors auf dem Retinoat. Dieselben Spektren werden beobachtet nach Wiederauflösung und nach folgender Wiederausgießung als Film. Die Daten sind in Tabelle 1 zusammengefaßt.

**Tabelle 1**

| | λ_{max,1} [nm] | λ_{max,2} [nm] | λ_{max,3} [nm] |
|---|---|---|---|
| Retionsäure methanolische Lösung | 348 | | |
| PDADMAC-Retinoat (Lösung) | 336 | | |
| PDADMAC-Retinoat (Film) | 319 | 252 | |
| Polyionen-6,3-Retinoat (Lösung) | 337 | | |
| Polyionen-6,3-Retinoat (Film) | 297 | 253 | |
| PM4VP-Lösung | - | 257 | 226 |
| PM4VP-Retinoat (Lösung) | 340 | 257 | 226 |
| PM4VP-Retinoat (Film) | 334 | 262 | 226 |

### Beispiel 6

### Herstellung von Komplexen der Vitamin A-Säure mit kationischen Polyaminosäuren

Polyaminosäuren sind Polyelektrolyte, die im Gegensatz zu anderen erfindungsgemäß verwendbaren Polyelektrolyten biologisch leicht abbaubar sind. Dies kann für verschiedene Anwendungsgebiete besonders vorteilhaft sein. Die Herstellung von bevorzugten Poly-L-Aminosäure Retinoat-Komplexen ist im Folgenden dargestellt. Die Herstellung von Polyethylenimin-Retinoat-Komplexen kann entsprechend durchgeführt werden.

### 6.1 Poly-L-lysin Retinoat

50 mg (0,24 mmol) Poly-L-lysin · HBr (Sigma/Aldrich) wurden in 20 ml entmineralisiertem Wasser gelöst und mit 10 %iger Natronlauge auf pH 9 eingestellt. Anschließend wurden 71,8 mg (0,24 mmol) Vitamin A-Säure (Fluka) in 50 ml mit Natronlauge auf pH 9 eingestelltem und in entmineralisiertem Wasser gelöst. Die Lösung von Poly-L-lysin wurde daraufhin langsam unter Rühren zu der Vitamin A-Säure-Lösung hinzugegeben. Es entstand eine klare, schwach gelbe Lösung, die eine Stunde weiter gerührt wurde. Anschließend wurde diese Lösung in eine Abdampfschale gegeben und, vor Lichteinwirkung geschützt, stehengelassen, bis das Wasser verdampft war. Man erhielt einen hellbraunen Film. Dieser Film wurde im Mörser zerrieben und das erhaltene Pulver zur Entfernung von überschüssigem NaBr mehrfach mit entmineralisiertem Wasser gewaschen und anschließend an der Luft getrocknet. Die Elementaranalyse ergab folgende Zusammensetzung des Komplexes (in Prozent):

| C₂₆H₄₁N₂O₃ | | | | | | |
|---|---|---|---|---|---|---|
| berechnet | C 72,7 | H 9,5 | N 6,5 | O 11,2 | Na - | Br - |
| gefunden | C 71,3 | H 9,7 | N 6,3 | O 12,7 | Na - | Br - |

Diese Ergebnisse belegen eine stöchiometrische 1 : 1 Komplexierung *6.2 Poly-L-histidin Retinoat*
78,4 mg (0,26 mmol) Vitamin A-Säure wurden in 50 ml mit Natronlauge auf pH 8 eingestelltem, entmineralisiertem Wasser gelöst. Anschließend wurde eine Lösung von 100 mg (0,52 mmol) Poly-L-histidin · HCI zugegeben, wobei ein feiner, hellgelber Niederschlag entstand. Der Niederschlag wurde abzentrifugiert und mehrfach mit entmineralisiertem Wasser gewaschen. Die Elementaranalyse ergab folgende Zusammensetzung des Komplexes (in Prozent):

| C₂₆H₃₆N₃O₃ | | | | | | |
|---|---|---|---|---|---|---|
| berechnet | C 71,2 | H 8,2 | N 9,6 | O 11,0 | Na - | Br - |
| gefunden | C 70,4 | H 8,6 | N 9,7 | O 11,3 | Na - | Br - |

Diese Ergebnisse belegen eine stöchiometrische 1 : 1 Komplexierung.

### 6.3 Poly-L-arginin Retinoat

Die Lösungen von 71,3 mg (0,24 mmol) Vitamin A-Säure in 50 ml entmineralisiertem Wasser und 50 mg (0,24 mmol) Poly-L-arginin HCl (Sigma/Aldrich) in 20 ml entmineralisiertem Wasser wurden mit Natronlauge auf pH 10 eingestellt. Unter starkem Rühren wurde die Polyelektrolytlösung zur Vitamin A-Säure zugetropft. Es bildete sich spontan ein hellgelber, flockiger Niederschlag. Die Lösung wurde noch ca. eine Stunde gerührt und der Niederschlag anschließend abzentrifugiert. Nach mehrfachem Waschen mit entmineralisiertem Wasser wurde das erhaltene Pulver an der Luft getrocknet. Die Elementaranalyse ergab folgende Zusammensetzung des Komplexes (in Prozent):

| C₂₆H₄₁N₄O₃ | | | | | | |
|---|---|---|---|---|---|---|
| berechnet | C 68,2 | H 9,0 | N 12,2 | O 10,5 | Na - | Br - |
| gefunden | C 68,4 | H 8,7 | N 12,7 | O 10,2 | Na - | Br - |

Diese Ergebnisse belegen eine stöchiometrische 1 : 1 Komplexierung.

### Beispiel 7

### Herstellung von Nano-Dispersionen aus Vitamin A-Säure-Komplexen

Für intravenöse Anwendungen von Arzneistoffen ist es beispielsweise notwendig, daß die arzneimittelhaltigen Teilchen in Emulsionen und Dispersionen ausreichend feinteilig sind. Als Obergrenze für die Teilchendurchmesser wird in der Regel 5000 nm angegeben. Im Folgenden wird gezeigt, daßdie Herstellung von stabilen Nano-Dispersionen aus erfindungsgemäßen Vitamin A-Säure-Komplexen in einfacher Weise möglich ist. Erfindungsgemäße Nano-Dispersionen aller in den Beispielen beschriebenen Komplexe können nach demselben Verfahren erhalten werden.

### 7.1 Herstellung einer Nano-Dispersion

Es wurden gleiche Mengen eines erfindungsgemäßen Vitamin A-Säure-Komplexes (20 mg eines Poly-L-lysin Retinoats) und des Dispergierhilfsmittels Poloxamer 188 (20 mg) (ICI Surfactants, (EO)₇₆-co-(PO)₃₀, M_{w} = 8350) mit einem Mörser fein verrieben. Das entstandene Pulver wurde im Anschluß daran in kleinen Portionen und unter starkem Rühren in je 15 ml entmineralisiertes Wasser gegeben, und die so hergestellte Rohdispersion wurde 15 mal je eine Minute mit Ultraschall behandelt. Dabei sollte die Dispersion nicht über 30°C erwärmt werden, da sonst eine chemische Zersetzung der Vitamin A-Säure zu befürchten ist. Nachfolgend wurde die Dispersion durch einen Filter (5 µm) gereinigt, um die erfindungsgemäße Nano-Dispersion zu erhalten.

### 7.2 Charakterisierung der Nano-Dispersionen

### a. Teilchengrößenbestimmung

Die Teilchengrößen der erfindungsgemäßen Nano-Dispersionen wurden mit dem "Submicron Particle Sizer, Model 370, Version 5,0" der Firma Nicomp erhalten. Dieses Gerät nutzt die Methode der dynamischen Lichtstreuung zur Ermittlung der Teilchengrößen im Bereich von 5 bis 5000 nm.

**Tabelle 2**

| Nach Intensität gewichtete, mittlere Teilchendurchmesser der Poly-L-Aminosäure-Retinoat Komplexe der Nano-Dispersionen | |
|---|---|
| | mittlerer Teilchenndurchmesser [nm] |
| Poly-L-lysin Retinoat | 1422 |
| Poly-L-arginin Retinoat | 378 |
| Poly-L-histidin Retinoat | 362 |

Die Teilchendurchmesser zeigten bei Lagerung der Nano-Dispersionen bei 10°C über einen Zeitraum von mindestens 3 Monaten keine wesentlichen Veränderungen.

Die Teilchengrößen liegen in den bevorzugten Ausführungsformen vorteilhafterweise weit unterhalb des als pharmakologisch für intravenöse Applikationen als kritisch angesehenen Grenzwertes von 5000 nm. Aufgrund der erfindungsgemäßen Teilchengrößen und Stabilität sind die erfindungsgemäßen Nano-Dispersionen der Vitamin A-Säure-Komplexe für intravenöse Anwendungen gut geeignet.

### b. Infrarotspektroskopie

Zum Nachweis intakter Komplexe in den hergestellten Nano-Dispersionen wurden diese IR-spektroskopisch untersucht (FT-IR Gerät Impact 400, Nicolet Instrument Corp.)

Wie Tabelle 3 zeigt, fehlt bei den Nano-Dispersionen der Poly-L-Aminosäure-Retinoat Komplexe die typische Carbonyl-Schwingungsbande bei 1690 cm⁻¹. Statt dessen wird mindestens eine weitere Bande bei ca. 1645 cm⁻¹ gefunden, die typisch für die komplexierte Vitamin A-Säure ist. Somit liegt in den erfindungsgemäßen Nano-Dispersionen keine freie Vitamin A-Säure vor.

**Tabelle 3**

| Charakteristische IR-Bandenlagen der Poly-L-Aminosäure-Retinoat Komplexe der Nano-Dispersionen | | | |
|---|---|---|---|
| Probe | 1. Signal [cm⁻¹] | 2. Signal [cm⁻¹] | 3. Signal |
| Poly-L-lysin Retinoat | 1645 | - | - |
| Poly-L-arginin Retinoat | 1639 | 1662 | - |
| Poly-L-histidin Retinoat | 1643 | 1661 | - |
| Vitamin A-Säure (in Ethanol) | - | - | 1690 |

### c. UV-Spektroskopie

Zum Nachweis des Vorliegens von intakten Komplexen in den hergestellten Nano-Dispersionen wurden UV-Spektren (UVIKON 931, Kontron Instruments) aufgenommen (vgl. Beispiel 5, Tabelle 1). Die komplexierte Vitamin A-Säure ist daran erkennbar, daß die Absorptionsbande gegenüber der freien Vitamin A-Säure einen deutlichen hypsochromen Shift (Blauverschiebung, Verschiebung zu kleineren Wellenlängen) und/oder eine weitere Banden bei geringeren Wellenlängen aufweist.

**Tabelle 3**

| UV-Absorptionsmaxima der Poly-L-Aminosäure-Retinoat Komplexe der Nano-Dispersionen | | |
|---|---|---|
| Probe | 1. Abs.-bande [nm] | 2. Abs.-bande [nm] |
| Poly-L-lysin Retinoat | - | 289 |
| Poly-L-arginin Retinoat | 334 | 286 |
| Poly-L-histidin Retinoat | 330 | 289 |
| Vitamin A-Säure (in Ethanol) | 348 | - |

## Patentansprüche

1. Mesomorpher Komplex aus Vitamin A-Säure und kationischen Polyelektrolyten.

2. Komplex nach Anspruch 1,
**dadurch gekennzeichnet,**
**dass** der Polyelektrolyt PDADMAC, Poly(ionen-6,3), PM4VP, Polyethylenimin oder eine Poly-L-Aminosäure ist.

3. Komplex nach Anspruch 2,
**dadurch gekennzeichnet,**
**dass** die Poly-L-Aminosäure Poly-L-arginin, Poly-L-histidin, Poly-L-lysin oder ein Gemisch davon ist.

4. Komplex nach einem der Ansprüche 1 bis 3,
**dadurch gekennzeichnet,**
**dass** er bis zu 70 % (w/w) Vitamin A-Säure enthält.

5. Komplex nach einem der vorhergehenden Ansprüche,
**dadurch gekennzeichnet,**
**dass** die Vitamin A-Säure und der kationische Polyelektrolyt in einem Verhältnis von 1 : 1 vorliegen,

6. Komplex nach einem der vorhergehenden Ansprüche,
**dadurch gekennzeichnet,**
**dass** er in Form eines viskoelastischen Films vorliegt.

7. Komplex nach einem der Ansprüche 1 bis 6,
**dadurch gekennzeichnet,**
**dass** er in einer Nano-Dispersion zusammen mit einem Dispergierhilfsmittel als Teilchen vorliegt, wobei der Teilchendurchmesser ≤ 5000 nm beträgt.

8. Komplex nach Anspruch 7,
**dadurch gekennzeichnet,**
**dass** das Dispergierhilfsmittel Poloxamer 188 ist.

9. Komplex nach Anspruch 7 oder 8,
**dadurch gekennzeichnet,**
**dass** der Komplex und das Dispergierhilfsmittel in gleichen Mengen enthalten sind.

10. Komplex nach einem der Ansprüche 7 bis 9,
**dadurch gekennzeichnet,**
**dass** der Teilchendurchmesser 200 bis 5000 nm, vorzugsweise 250 bis 3000 nm, besonders bevorzugt 300 bis 2000 nm und am meisten bevorzugt 350 bis 1500 nm beträgt.

11. Verfahren zur Herstellung mesomorpher Komplexe aus Vitamin A-Säure und kationischen Polyelektrolyten,
**dadurch gekennzeichnet,**
**dass** man Lösungen von Vitamin A-Säure und eines Polyelektrolyten mischt, die gebildeten Rohkomplexe isoliert und gegebenenfalls nach an sich bekannten Methoden aufreinigt.

12. Verfahren nach Anspruch 11,
**dadurch gekennzeichnet,**
**dass** man als Polyelektrolyten PDADMAC, PM4VP, Poly(ionen-6,3-bromid oder -chlorid), Polyethylenimin oder Poly-L-Aminosäuren verwendet.

13. Verfahren nach Anspruch 12,
**dadurch gekennzeichnet,**
**dass** man als Poly-L-Aminosäure Poly-L-arginin, Poly-L-histidin, Poly-L-lysin oder ein Gemisch davon verwendet.

14. Verfahren nach einem der Ansprüche 11 oder 13,
**dadurch gekennzeichnet,**
**dass** man in basischer Lösung arbeitet.

15. Verwendung von mesomorphen Komplexen nach einem der Ansprüche 1 bis 10 als Vitamin A-Substituat zur Herstellung von Arzneimitteln.

16. Verwendung von mesomorphen Komplexen nach einem der Ansprüche 1-12
zur Herstellung von Arzneimitteln zur Behandlung von Hautkrankheiten oder zur Hemmung des Wachstums maligner Tumoren.

17. Pharmazeutische Zusammensetzung,
**dadurch gekennzeichnet,**
**dass** sie mindestens einen mesomorphen Komplex nach einem der Ansprüche 1 bis 10 enthält.

## Claims

1. Mesomorphic complex of vitamin A acid and cationic polyelectrolytes.

2. Complex according to claim 1, **characterised in that** the polyelectrolyte is PDADMAC, poly(ionene-6,3), PM4VP, polyethyleneimine or a poly-L-amino acid.

3. Complex according to claim 2, **characterised in that** the poly-L-amino acid is poly-L-arginine, poly-L-histidine, poly-L-lysine or a mixture thereof.

4. Complex according to any of claims 1 to 3, **characterised in that** it contains up to 70% (w/w) vitamin A acid.

5. Complex according to any of the preceding claims, **characterised in that** the vitamin A acid and the cationic polyelectrolyte are present in a ratio of 1:1.

6. Complex according to any of the preceding claims, **characterised in that** it is in the form of a viscoelastic film.

7. Complex according to any of claims 1 to 6, **characterised in that** it is present as particles in a nanodispersion together with a dispersing aid, the particle diameter being ≤ 5,000 nm.

8. Complex according to claim 7, **characterised in that** the dispersing aid is poloxamer 188.

9. Complex according to claim 7 or 8, **characterised in that** the complex and the dispersing aid are present in equal amounts.

10. Complex according to any of claims 7 to 9, **characterised in that** the particle diameter is 200 to 5,000 nm, preferably 250 to 3,000 nm, particularly preferably 300 to 2,000 nm and most preferably 350 to 1,500 nm.

11. Process for preparing mesomorphic complexes of vitamin A acid and cationic polyelectrolytes, **characterised in that** solutions of vitamin A acid and of a polelectrolyte are mixed, the crude complexes which have formed are isolated and are optionally purified by methods known *per se.*

12. Process according to claim 11, **characterised in that** PDADMAC, PM4VP, poly(ionene-6,3 bromide or chloride), polyethyleneimine or poly-L-amino acids are used as polyelectrolytes.

13. Process according to claim 12, **characterised in that** poly-L-arginine, poly-L-histidine, poly-L-lysine or a mixture thereof is used as poly-L-amino acid.

14. Process according to either of claims 11 or 13, **characterised in that** a basic solution is used.

15. Use of mesomorphic complexes according to any of claims 1 to 10 as vitamin A substitute to produce pharmaceutical compositions.

16. Use of mesomorphic complexes according to any of claims 1 to 16 to produce pharmaceutical compositions for treating skin disorders or for inhibiting the growth of malignant tumors.

17. Pharmaceutical composition **characterised in that** it contains at least one mesomorphic complex according to any of claims 1 to 10.

## Revendications

1. Complexe mésomorphe d'acide rétinoïque et de polyélectrolytes cationiques.

2. Complexe selon la revendication 1, **caractérisé en ce que** le polyélectrolyte est un PDADMAC, un poly(6,3-ionène), un PM4VP, une polyéthylène-imine ou un poly(L-aminoacide).

3. Complexe selon la revendication 2, **caractérisé en ce que** le poly(L-aminoacide) est une poly(L-arginine), une poly(L-histidine), une poly(L-lysine) ou un de leurs mélanges.

4. Complexe selon l'une des revendications 1 à 3, **caractérisé en ce qu'**il contient jusqu'à 70 % en masse d'acide rétinoïque.

5. Complexe selon l'une des revendications précédentes, **caractérisé en ce que** le rapport de l'acide rétinoïque et du polyélectrolyte cationique est de 1:1.

6. Complexe selon l'une des revendications précédentes, **caractérisé en ce qu'**il est sous forme d'un film viscoélastique.

7. Complexe selon l'une des revendications 1 à 6, **caractérisé en ce qu'**il se trouve sous forme de particules avec un auxiliaire de dispersion dans une nanodispersion dans laquelle le diamètre des particules est inférieur ou égal à 5 000 nm.

8. Complexe selon la revendication 7, **caractérisé en ce que** l'auxiliaire de dispersion est le poloxamère 188.

9. Complexe selon la revendication 7 ou 8, **caractérisé en ce que** le complexe et l'auxiliaire de dispersion sont contenus en les mêmes quantités.

10. Complexe selon l'une des revendications 7 à 9, **caractérisé en ce que** le diamètre des particules est de 200 à 5 000 nm, de préférence de 250 à 3 000 nm, de façon particulièrement préférée de 300 à 2 000 nm et au mieux de 350 à 1 500 nm.

11. Procédé de préparation de complexes mésomorphes d'acide rétinoïque et de polyélectrolytes cationiques, **caractérisé en ce que** l'on mélange des solutions d'acide rétinoïque et d'un polyélectrolyte, on isole les complexes bruts formés et on les purifie éventuellement par des procédés connus en soi.

12. Procédé selon la revendication 11, **caractérisé en ce que** l'on utilise comme polyélectrolyte un PDADMAC, un PM4VP, un poly(bromure ou chlorure de 6,3-ionène), une polyéthylène-imine ou un poly(L-aminoacide).

13. Procédé selon la revendication 12, **caractérisé en ce que** l'on utilise comme poly(L-aminoacide) une poly(L-arginine), une poly(L-histidine), une poly(L-lysine) ou un de leurs mélanges.

14. Procédé selon l'une des revendications 11 ou 13, **caractérisé en ce que** l'on travaille en solution basique.

15. Utilisation de complexes mésomorphes selon l'une des revendications 1 à 10 comme substitut de la vitamine A pour la préparation de médicaments.

16. Utilisation de complexes mésomorphes selon l'une des revendications 1 à 10 pour la préparation de médicaments destinés au traitement de maladies de la peau ou à l'inhibition de la croissance de tumeurs malignes.

17. Composition pharmaceutique **caractérisée en ce qu'**elle contient au moins un complexe mésomorphe selon l'une des revendications 1 à 10.
